# EUROPÄISCHE PATENTSCHRIFT

(11) **EP 2 736 586 B1**
(45) Veröffentlichungstag und Bekanntmachung des Hinweises auf die Patenterteilung: **27.11.2019**
(21) Anmeldenummer: 12740503.3
(22) Anmeldetag: 27.07.2012
(51) Int. Cl.: A61M 39/28, A61M 1/36, A61M 39/12, A61M 39/16, A61M 39/20

(54) **MEDIZINISCHER SCHLAUCH SOWIE VERFAHREN ZUM STERILISIEREN EINES MEDIZINISCHEN SCHLAUCHS**
MEDICAL TUBE AND METHOD FOR STERILIZING A MEDICAL TUBE
TUBE MÉDICAL ET PROCÉDÉ DE STÉRILISATION D'UN TUBE MÉDICAL

(30) Priorität: 29.07.2011 DE 102011110474; 29.07.2011 US 201161512958 P
(43) Veröffentlichungstag der Anmeldung: 04.06.2014
(73) Patentinhaber: Fresenius Medical Care Deutschland GmbH, 61352 Bad Homburg (DE)
(72) Erfinder: HAECKER, Juergen, 61267 Neu-Anspach (DE); LEICK, Lothar, 66663 Merzig-Ballern (DE); SCHULZ, Wolfgang, 66606 St. Wendel (DE); THIRY, Edmund-Jakob, 66636 Tholey (DE)
(74) Vertreter: Bobbert & Partner Patentanwälte PartmbB
(86) Internationale Anmeldenummer: PCT/EP2012/003191
(87) Internationale Veröffentlichungsnummer: WO 2013/017237

(56) Entgegenhaltungen:
- EP-A2- 0 186 509
- EP-A2- 1 905 478
- WO-A1-98/48872
- WO-A2-03/030962
- US-A1- 2005 253 390
- US-A1- 2007 225 684

## Beschreibung

Die vorliegende Erfindung betrifft einen medizinischen Schlauch gemäß Anspruch 1 sowie ein Verfahren zum Sterilisieren eines medizinischen Schlauchs gemäß Anspruch 9.

Aus der Praxis sind mechanisch wirkende Schlauchklemmen für medizinische Schläuche wie Infusionsleitungen, Blutkreisläufe, aber auch für Schläuche für den Einsatz im Labor bekannt. Derartige Schlauchklemmen liegen außen am Schlauch an. Sie können manuell in eine offene Stellung, in welcher sie den Fluidstrom im Schlauch nicht behindern, oder in eine geschlossene Stellung, in welcher sie den Fluidstrom beispielsweise dadurch mindern oder unterbinden, dass sie den Schlauch quetschen oder klemmen, gebracht werden.

Schlauchklemmen dienen somit als mechanische Steuerungssysteme für den Durchfluss von medizinischen Fluiden durch den Schlauch, beispielsweise von Blut, Infusionslösungen wie isotonische Lösungen, und Medikamenten.

Aus der EP 1 905 478 A2 ist eine Katheteranordnung mit geringem Profil bekannt.

Aus der WO 03/030962 A2 ist ein Ring zum Versehen eines Katheters mit Informationen bekannt.

US 2007/225684 A1 ist eine Luer Konnektoranordnung mit Schlauchklemme bekannt. Die Merkmale des Oberbegriffs des Ansprunchs 1 sind in diesem Dokument offenbart.

Aus der WO 98/48872 A1 ist ein aseptischer, weiblicher Verbinder bekannt.

Aus der WO 2005/253390 A1 ist eine rasch zu lösende, medizinische Schlauchverbindung bekannt.

Aus der EP 0 186 509 A2 ist ein Clip zum Verschließen eines Schlauchlumens bekannt.

Eine Aufgabe der vorliegenden Erfindung ist es, einen Schlauch mit einer Haltevorrichtung zum Halten wenigstens einer Schlauchklemme an einem medizinischen Schlauch vorzuschlagen. Zudem soll ein Verfahren zum Sterilisieren eines erfindungsgemäßen medizinischen Schlauchs angegeben werden.

Die erfindungsgemäße Aufgabe wird durch einen medizinischen Schlauch mit den Merkmalen des Anspruchs 1 sowie ein Verfahren mit den Merkmalen des Anspruchs : 9 gelöst.

Erfindungsgemäß wird somit der Schlauch des Anspruchs 1 mit einer Haltevorrichtung zum Halten wenigstens einer Schlauchklemme an einem medizinischen Schlauch vorgeschlagen, wobei die Schlauchklemme wenigstens einen, zwei oder mehr Klemmabschnitte aufweist zum Verändern eines Lumenquerschnitts eines medizinischen Schlauchs im Gebrauch. Die Haltevorrichtung weist wenigstens eine Aufnahmestruktur zum lösbaren Aufnehmen wenigstens eines Abschnitts der Schlauchklemme auf.

Das erfindungsgemäße Verfahren nach Anspruch 9 dient dem Sterilisieren eines medizinischen Schlauchs, welcher wenigstens eine Schlauchklemme aufweist, welche wenigstens zwei Klemmabschnitte zum Verändern eines Lumenquerschnitts des medizinischen Schlauchs hat. Der Schlauch hat wenigstens eine Haltevorrichtung - welche insbesondere ausgestaltet ist gemäß einer hierin beschriebenen Ausführungsform -, welche wenigstens eine Aufnahmestruktur zum lösbaren Aufnehmen wenigstens eines Abschnitts der Schlauchklemme aufweist. Das Verfahren umfasst das Verbringen der Schlauchklemme in eine Parkposition an der Haltevorrichtung, in welcher der aufgenommene Abschnitt der Schlauchklemme in oder an der Aufnahmestruktur angeordnet ist. Ferner umfasst das Verfahren das gemeinsame Sterilisieren von Schlauch und der in die Parkposition verbrachten Schlauchklemme.

Weiterentwicklungen der vorliegenden Erfindung sind jeweils Gegenstand von Unteransprüchen und Ausführungsformen. Erfindungsgemäße Ausführungsformen können eines oder mehrere der im Folgenden genannten Merkmale aufweisen, solange diese Ausführungsformen der beanspruchten Erfindung entsprechen.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

In bestimmten erfindungsgemäßen Ausführungsformen sind die ausgestalteten Klemmabschnitte gleichartig. In anderen erfindungsgemäßen Ausführungsformen sind wenigstens ein erster Klemmabschnitt und ein zweiter Klemmabschnitt voneinander verschieden ausgestaltet.

Das Verändern eines Lumenquerschnitts eines medizinischen Schlauchs ist in manchen erfindungsgemäßen Ausführungsformen ein Abklemmen oder Abquetschen. Dies kann einen Volumenstrom für ein medizinisches Fluid entlang des Lumens des Schlauchs zumindest verringern oder vollständig unterbinden oder verhindern.

In bestimmten erfindungsgemäßen Ausführungsformen ist oder umfasst das lösbare Aufnehmen wenigstens eines Abschnitts der Schlauchklemme ein Eingreifen und/oder ein Verhaken und/oder Verklemmen und/oder Verschnappen.

Die Haltevorrichtung ist in einigen Ausführungsformen ein Schlauchkonnektor oder weist wenigstens einen solchen auf.

Der Schlauchkonnektor ist in einigen erfindungsgemäßen Ausführungsformen als ein arterieller und/oder venöser Patientenkonnektor eines extrakorporalen Blutschlauchsatzes ausgestaltet, insbesondere als ein solcher, an welchen jeweils die arterielle und/oder die venöse Nadel angeschlossen wird.

Unter einer Nadel wird in bestimmten erfindungsgemäßen Ausführungsformen die Patientennadel samt Punktionsflügel und Schlauchstück und entsprechender Konnektorhälfte verstanden.

In manchen Ausführungsformen ist die Haltevorrichtung ein Schlauchaufsatz, oder sie weist wenigstens einen solchen auf.

Der Schlauchkonnektor und/oder der Schlauchaufsatz sind im Gebrauch des Schlauchs in bestimmten erfindungsgemäßen Ausführungsformen nicht längs zu diesem verschiebbar.

Haltevorrichtung, Schlauchkonnektor und/oder Schlauchaufsatz sind in gewissen erfindungsgemäßen Ausführungsformen ein einstückiges oder ein nicht-einstückiges Bauteil einer Blutkassette oder eines Schlauchsatzes.

Erfindungsgemäß ist der Abschnitt der Schlauchklemme, welcher lösbar von der Aufnahmestruktur aufgenommen wird, wenigstens ein Klemmabschnitt, oder er weist wenigstens einen solchen auf.

In bestimmten erfindungsgemäßen Ausführungsformen bedeutet das lösbare Aufnehmen der Schlauchklemme oder eines Abschnitts hiervon an der Haltevorrichtung, dass die gesamte Schlauchklemme im Gebrauch des Schlauchs (oder zu seinem Gebrauch) vollständig von der Haltevorrichtung getrennt werden kann. Dies ist in einigen erfindungsgemäßen Ausführungsformen dadurch möglich, dass die Schlauchklemme entlang des Schlauchs von der Haltevorrichtung heruntergeschoben oder -gedrückt wird.

Die Aufnahmestruktur ist in einigen erfindungsgemäßen Ausführungsformen geometrisch derart an einen Abschnitt der Schlauchklemme angepasst, beispielsweise an die vorhandenen Klemmabschnitte, dass die Schlauchklemme während des Sterilisationsprozesses oder während der Lagerung jeweils in der Parkposition verbleibt und/oder sich ohne fremdes Zutun zumindest nicht entlang des Schlauchs bewegen kann.

Erfindungsgemäß ist die Haltevorrichtung und/oder ihre Aufnahmestruktur vorgesehen, ein Verändern des Lumenquerschnitts des Schlauchs, insbesondere in dessen flexiblem Leitungsabschnitt, mittels der Schlauchklemme zu verhindern, solange sich diese in der Parkposition befindet.

In manchen erfindungsgemäßen Ausführungsformen der Haltevorrichtung ist die Aufnahmestruktur als wenigstens eine um die Haltevorrichtung - um deren gesamten Umfang oder um nur einen oder mehrere Abschnitte des Umfangs - umlaufende Aussparung ausgestaltet, oder sie weist wenigstens eine solche auf.

In bestimmten erfindungsgemäßen Ausführungsformen ist die Aufnahmestruktur der Haltevorrichtung gegenüber der Haltevorrichtung abgegrenzt.

In manchen erfindungsgemäßen Ausführungsformen unterscheidet sich der Abschnitt des Schlauchs, welcher die Haltevorrichtung trägt, konstruktiv von wenigstens einem hierzu benachbarten Abschnitt des Schlauchs.

In bestimmten Ausführungsformen der Haltevorrichtung weist diese einen Verdrehschutz auf. Dieser erlaubt ein Verdrehen der Schlauchklemme um eine Längsachse der Haltevorrichtung nur in eingeschränktem Maße oder unterbindet ein solches Verdrehen vollständig.

Ein solcher Verdrehschutz kann Anschläge, Begrenzungen, Stopps, Rillen, Nuten, Erhebungen und dergleichen aufweisen.

In einigen erfindungsgemäßen Ausführungsformen ist die Aussparung der Aufnahmestruktur eine Nut oder eine Rille oder sie weist wenigstens eine Nut oder wenigstens eine Rille auf.

In manchen Ausführungsformen der Haltevorrichtung ist die Aufnahmestruktur wenigstens eine um die Haltevorrichtung - um deren gesamten Umfang oder um nur einen oder mehrere Abschnitte des Umfangs - umlaufende Erhebung oder weist wenigstens eine solche auf.

Die Aussparung und/oder die Erhebung sind in bestimmten erfindungsgemäßen Ausführungsformen - insbesondere vollständig oder im Wesentlichen - ringförmig.

Mit dem Begriff ringförmig werden in manchen erfindungsgemäßen Ausführungsformen nicht nur entlang eines Umfangs der Haltevorrichtung geschlossene Formen verstanden. Vielmehr zählen hierzu auch Formen, welche eine oder mehrere Unterbrechungen aufweisen.

In gewissen erfindungsgemäßen Ausführungsformen weist die Aufnahmestruktur eine Kombination von Aussparung(en) und Erhebung(en) auf. Sie kann beispielsweise zwei Erhebungen und eine Aussparung aufweisen. Diese können beispielsweise in der Folge Erhebung, Aussparung, Erhebung in Längsrichtung der Haltevorrichtung oder Schlauchlängsrichtung hintereinander angeordnet sein.

Erfindungsgemäß weist die Haltevorrichtung wenigstens eine Schlauchklemme auf. Sie kann auch beispielsweise zwei oder mehr Schlauchklemmen aufweisen. Weist sie mehrere Schlauchklemmen auf, so kann sie auch mehrere Aufnahmestrukturen aufweisen.

Die Haltevorrichtung ist oder wurde in manchen Ausführungsformen zusammen oder gemeinsam mit der Schlauchklemme sterilisiert.

Die Haltevorrichtung kann ganz oder teilweise aus demselben Material wie der Schlauch hergestellt sein. In bestimmten erfindungsgemäßen Ausführungsformen ist die Haltevorrichtung aus einem härteren Material als der Schlauch oder als dessen - zumeist flexible, was hier als biegsam und/oder als mittels der Schlauchklemme komprimierbar zu verstehen ist - Leitungsabschnitte hergestellt.

Insbesondere sind in bestimmten Ausführungsformen des erfindungsgemäßen medizinischen Schlauchs die Haltevorrichtung oder wenigstens ein Abschnitt hiervon aus einem ersten Material hergestellt oder weisen ein solches auf. Der Schlauch oder wenigstens ein Abschnitt des Schlauchs ist aus einem zweiten Material hergestellt oder weist ein solches auf. Das erste und das zweite Material unterscheiden sich. Der Abschnitt des Schlauchs kann insbesondere ein flexibler Leitungsabschnitt sein, welcher im Gebrauch mittels der Schlauchklemme geklemmt werden kann oder soll.

In manchen erfindungsgemäßen Ausführungsformen ist das erste Material der Haltevorrichtung härter oder druckfester als das zweite Material des Schlauchs.

In gewissen erfindungsgemäßen Ausführungsformen ist die Schlauchklemme während der Benutzung des Schlauchs axial entlang des Schlauchs verschiebbar, vorzugsweise frei verschiebbar. In einigen erfindungsgemäßen Ausführungsformen ist die Schlauchklemme an einer Vielzahl von Abschnitten des Schlauchs bestimmungsgemäß, d. h. zum Verändern oder Sperren des Lumens des Schlauchs, verwendbar, vorzugsweise unter beliebigem Abstand zur Haltevorrichtung.

In manchen erfindungsgemäßen Ausführungsformen ist die Schlauchklemme ohne Zutun oder Wechselwirkung mit der Haltevorrichtung bestimmungsgemäß verwendbar.

In bestimmten erfindungsgemäßen Ausführungsformen ist wenigstens einer der Klemmabschnitte (oder alle) ausgestaltet und/oder angeordnet, um das Lumen des Schlauchs durch - vorzugsweise ausschließlich oder im Wesentlichen - radiales, vorzugweise unmittelbares, Einwirken der Klemme(n) auf den Schlauch zu verändern oder einen Durchfluss durch den Schlauch zu unterbinden.

In manchen erfindungsgemäßen Ausführungsformen ist die Haltevorrichtung keine Ventileinrichtung.

In gewissen erfindungsgemäßen Ausführungsformen ist die Haltevorrichtung nicht ausgestaltet, um bestimmungsgemäß zu einer Veränderung des Lumens des Schlauchs zu dienen und/oder hierzu beizutragen.

In manchen erfindungsgemäßen Ausführungsformen weist die Schlauchklemme keinen hiervon abstehenden Hebel auf.

In einigen erfindungsgemäßen Ausführungsformen ist der Schlauch ausgestaltet als extrakorporaler Blutkreislauf oder als ein Abschnitt hiervon.

In manchen erfindungsgemäßen Ausführungsformen befindet sich die Schlauchklemme während ihres Verbringens in eine Parkposition an der Haltevorrichtung in einem geöffneten Zustand.

In bestimmten erfindungsgemäßen Ausführungsformen ist das "gemeinsame" Sterilisieren von Schlauch und der in die Parkposition verbrachten Schlauchklemme als zeitgleich und/oder mit demselben Sterilisationsverfahren durchgeführt zu verstehen. In einigen erfindungsgemäßen Ausführungsformen ist unter einem "gemeinsamen" Sterilisieren zu verstehen, dass die Schlauchklemme mit der Haltevorrichtung während des Sterilisierens verbunden ist.

Das erfindungsgemäße Verfahren umfasst in einigen Ausführungsformen das Verbringen wenigstens eines Klemmabschnitts in oder an die Aufnahmestruktur, vor oder zum Sterilisieren des medizinischen Schlauchs.

Die Haltevorrichtung kann einstückig mit den flexiblen Leitungsabschnitten des Schlauchs ausgestaltet sein. Sie kann aber auch ein separat hergestelltes Element sein, das anschließend mit den flexiblen Leitungsabschnitten des Schlauchs verbunden wurde.

In Ausführungsformen, in welchen die Haltevorrichtung und die übrigen Abschnittes des Schlauchs separate Teile sind, kann vorgesehen sein, dass Abschnitte des Schlauchs, insbesondere flexible, leitende Abschnitte, oder ein Endabschnitt hiervon nur soweit in ein Inneres der Haltevorrichtung eingeführt ist/sind, dass dieser nicht unter die Aufnahmestruktur und/oder nicht unter eine Aussparung für die Klemmabschnitte reicht. Bei dieser Ausgestaltung ist vorteilhaft sichergestellt, dass durch die Schlauchklemme ausgeübter Druck keinen Abschnitt des Schlauches beschädigen kann. Dies gilt selbst dann, wenn der Druck gewollt oder ungewollt sehr hoch sein sollte.

In einigen erfindungsgemäßen Ausführungsformen kann die Haltevorrichtung nicht entlang der Längsachse des Schlauchs bewegt werden.

Einige oder alle erfindungsgemäßen Ausführungsformen können einen oder mehrere der oben oder im Folgenden genannten Vorteile aufweisen.

In der geschlossenen Position übt die Schlauchklemme funktionsbedingt Druck über einen Abschnitt des Schlauches aus, um den gewünschten Fluiddurchfluss im Schlauch zu erreichen. Es ist aus der Praxis bekannt, dass dieser Druck den Schlauch jedenfalls nach einer gewissen Zeit beschädigen kann. Aber selbst in ihrer geöffneten Position können Schlauchklemmen den Schlauch druckbedingt beschädigen oder in seinem Querschnitt anhaltend verändern. Dies kann insbesondere während der gemeinsamen Sterilisation von Schlauch und Schlauchklemme und/oder während der sich hieran anschließenden Lagerung geschehen. Eine solche Beschädigung oder Veränderung des Schlauches kann zur Entstehung von Leckagen oder von Schlauchabschnitten, in welchen bei Gebrauch Turbulenzen auftreten und/oder die ein Abknicken des Schlauchs begünstigen, führen. Letzteres kann zur Bildung von Thromben, einem Verklumpen bestimmter Fluide, einem Verschluss des Schlauches usw. führen. Zudem kann die Materialbeschädigung hygienisch bedenklich sein, da in bestimmten Fällen die Schlauchschädigung einen Eintrittsbereich für Keime bilden kann. Derartige Schädigungen und die hiermit verbundenen Folgen können bei Einsatz der vorliegenden Erfindung vorteilhaft vermieden werden.

Die vorliegende Erfindung wird im Folgenden anhand der beigefügten Zeichnungen, in welcher identische Bezugszeichen gleiche oder ähnliche Bauteile bezeichnen, exemplarisch erläutert. Für die zum Teil vereinfachten Figuren gilt:
- **Fig. 1**: zeigt einen erfindungsgemäßen Schlauch mit einer Haltevorrichtung in einer Park- oder Sterilisationsstellung einer mit dem Schlauch verbundenen Schlauchklemme; und
- **Fig. 2**: zeigt die Anordnung der Fig. 1 in einer Gebrauchstellung des Schlauchs.

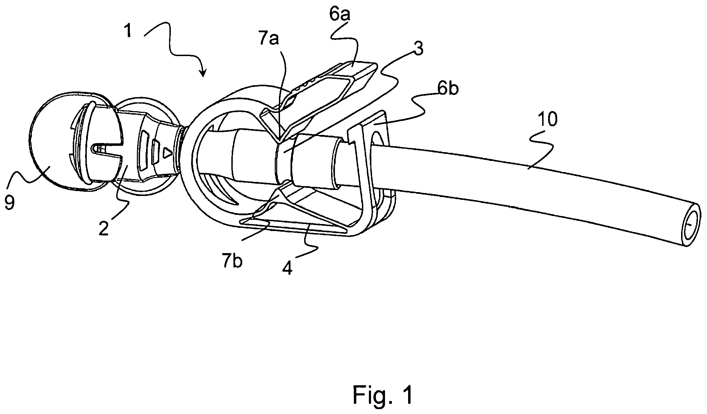

**Fig. 1** zeigt einen erfindungsgemäßen medizinischen Schlauch 10 mit einer Haltevorrichtung 1 und einem sich hieran (nach rechts in Fig. 1) anschließenden flexiblen Leitungsabschnitt.

Die Haltevorrichtung 1 der Fig. 1 ist rein exemplarisch als ein Schlauchkonnektor ausgeführt, mittels welchem der Schlauch 10 mit dem Gefäßsystem des Patienten oder mit einem medizinischen Gerät verbunden wird. Die Haltevorrichtung 1 weist zu diesem Zweck einen Konnektionsabschnitt 2 auf.

Die Haltevorrichtung 1 weist zudem eine Aufnahmestruktur 3 auf zum Aufnehmen einer Schlauchklemme 4 in einer Parkposition oder -stellung (welche hierin auch als Ruhe- oder Sterilisationsposition oder -stellung bezeichnet ist). Die Schlauchklemme 4 ist in Fig. 1 in einer solchen Parkstellung gezeigt. In dieser Parkstellung ist die Schlauchklemme 4, welche im Beispiel der Fig. 1 sowohl Teile der Haltevorrichtung 1 als auch einen Teil des flexiblen Leitungsabschnitts überdeckt, mit einem Abschnitt hiervon lösbar mit der Haltevorrichtung 1 verbunden. Die Schlauchklemme 4 weist zwei Schnappstücke 6a, 6b zum Schließen der Klemme 4 im Gebrauch auf. Ferner weist die Schlauchklemme 4 zwei Klemmabschnitte 7a, 7b auf. Im Beispiel der Fig. 1 ist die Schlauchklemme 4 mittels ihrer Klemmabschnitte 7a, 7b mit der Aufnahmestruktur 3 verbunden oder hierin aufgenommen. Fig. 1 zeigt ferner eine lösbare Verschlusskappe 9 für den Konnektionsabschnitt 2 zum Verhindern einer Kontamination der Haltevorrichtung 1 bis zum Gebrauch des Schlauches 10.

Der Schlauch 10 ist durch die Schlauchklemme 4 hindurch geführt. Die Schlauchklemme 4 umgreift somit den Schlauch 10. Die beiden einander gegenüberliegenden Klemmabschnitte 7a und 7b zum Klemmen eines zwischen ihnen liegenden Schlauchabschnittes greifen in die Aufnahmestruktur 3 ein.

Die Aufnahmestruktur 3 ist in Fig. 1 als eine ringförmige Aussparung ausgestaltet, d. h. eine Aussparung, welche sich um den Umfang der Haltevorrichtung 1 erstreckt. Der Querschnitt der Aussparung kann alle denkbaren Formen aufweisen, welche geeignet sind, wenigstens einen Abschnitt der Schlauchklemme 4 aufzunehmen oder zu begrenzen. Der Querschnitt der Aussparung kann beispielsweise V-förmig, U-förmig, etc. sein.

Da die Aufnahmestruktur 3 die Klemmabschnitte 7a, 7b der Schlauchklemme 4 aufnimmt, wenn die Schlauchklemme 4 in der in Fig. 1 gezeigten Parkstellung in der Aufnahmestruktur 3 ruht, verhindert die Aufnahmestruktur 3, dass sich die Schlauchklemme 4 während eines Sterilisationsprozesses oder während der anschließenden Lagerung eines Schlauches 10 entlang des Schlauches 10 ungehindert bewegen kann. Verhindert wird auf diese Weise auch, dass die Klemmabschnitte 7a, 7b oder ein anderer Abschnitt der Schlauchklemme 4 den Schlauch 10 ungewollt klemmen und gar durch Druck schädigen.

In Fig. 1 weist der Schlauch 10 genau eine Haltevorrichtung 1 auf. Es ist allerdings auch erfindungsgemäß vorgesehen, dass ein erfindungsgemäßer Schlauch mehrere Haltevorrichtungen aufweist. Ebenso ist vorgesehen, mittels einer Haltevorrichtung 1 bei komplizierteren Schlauchsystemen, beispielsweise Fluidleitungssystemen mit mehreren, parallel eingeschalteten Schläuchen 10, wie bei Zentralvenenkathetern der Fall, mehrere Schläuche abzuklemmen.

In der Ausgestaltung der Fig. 1 weist die als Konnektor ausgestaltete Haltevorrichtung 1 an ihrem Konnektionsabschnitt 2 einen Schraubmechanismus für ihre Verbindung auf. Alternativ oder ergänzend kann der Konnektionsabschnitt 2 jedes aus dem Stand der Technik bekannte Konnektionssystem oder jeden Konnektionsmechanismus aufweisen, beispielsweise einen Steck- oder einen Drehmechanismus.

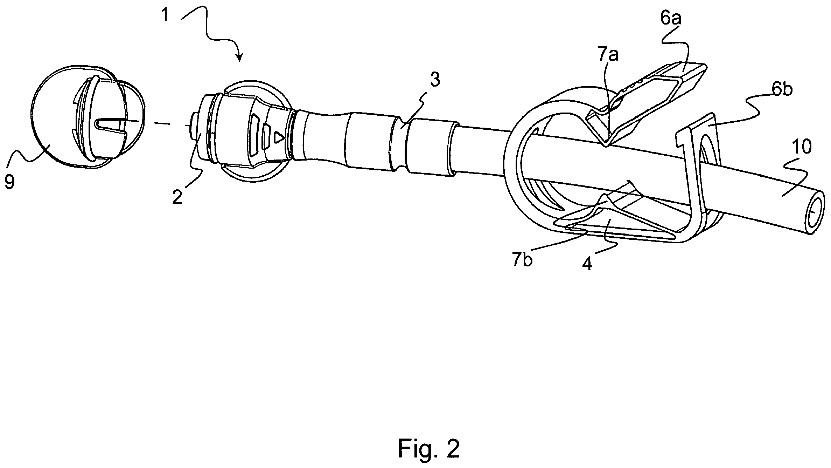

**Fig. 2** zeigt die Anordnung der Fig. 1 in einer Gebrauchsstellung des Schlauchs 10. Die Schlauchklemme 4 ist nicht mehr in der Parkstellung auf der Haltevorrichtung 1. Sie ist vielmehr zu einem Schlauchabschnitt hin verschoben, an welchem ihre Klemmwirkung gewünscht ist.

Die Verschlusskappe 9 ist in Fig. 2 losgelöst vom Konnektionsabschnitt 2 gezeigt. In diesem Zustand kann der Schlauch 10 beispielsweise mit einem venösen oder arteriellen Zugang eines Patienten, oder mit einem Konnektionsstück eines medizinischen Gerätes usw. verbunden werden.

Im Gebrauch werden die zwei Schnappstücke 6a, 6b der Schlauchklemme 4 miteinander verrastet. Die Klemmabschnitte 7a, 7b werden dann aufeinander zu gespannt und üben somit Druck auf den dazwischen eingeklemmten Schlauch 10 aus. Auf diese Weise verändert sich der Lumenquerschnitt des Schlauches 10. Der Durchfluss des im Schlauch 10 geleiteten Fluids variiert entsprechend dieser Lumenquerschnittsveränderung.

Die dargestellte Ausführungsform der Schlauchklemme 4 in beiden Figuren soll nur als Beispiel verstanden werden. Andere aus dem Stand der Technik bekannte Schlauchklemmen können selbstverständlich ebenfalls mittels der Haltevorrichtung 1 in einer Parkposition gehalten werden.

### verwendete Bezugszeichen

- 1: Haltevorrichtung als Schlauchkonnektor
- 2: Konnektionsabschnitt
- 3: Aufnahmestruktur
- 4: Schlauchklemme
- 6a, b: Schnappstück
- 7a, b: Klemmabschnitt
- 9: Verschlusskappe
- 10: Schlauch

## Patentansprüche

1. Medizinischer Schlauch (10) mit wenigstens einer Haltevorrichtung (1) mit Schlauchklemme (4), wobei die Haltevorrichtung (1) zum Halten der Schlauchklemme (4) an dem medizinischen Schlauch (10) ausgestaltet ist, wobei die Schlauchklemme (4) wenigstens einen Klemmabschnitt (7a, 7b) aufweist zum Verändern eines Lumenquerschnitts des medizinischen Schlauchs (10) im Gebrauch, wobei die Haltevorrichtung (1) wenigstens eine Aufnahmestruktur (3) zum lösbaren Aufnehmen wenigstens eines Abschnitts der Schlauchklemme (4) aufweist, wobei der Abschnitt der Schlauchklemme (4), welcher lösbar von der Aufnahmestruktur (3) aufgenommen wird, wenigstens ein vorgenannter Klemmabschnitt (7a,7b) ist oder einen solchen aufweist, **dadurch gekennzeichnet, dass** die Haltevorrichtung (1) und/oder ihre Aufnahmestruktur (3) vorgesehen ist, ein Verändern des Lumenquerschnitts des Schlauchs (10), insbesondere in dessen flexiblem Leitungsabschnitt, mittels der Schlauchklemme (4) zu verhindern, solange sich die Schlauchklemme (4) in der Parkposition, in welcher der Abschnitt der Schlauchklemme (4) in oder an der Aufnahmestruktur (3) angeordnet ist, befindet.

2. Medizinischer Schlauch (10) nach Anspruch 1, wobei die Haltevorrichtung (1) ein Schlauchkonnektor ist oder einen solchen aufweist.

3. Medizinischer Schlauch (10) nach Anspruch 1, wobei die Haltevorrichtung (1) ein Schlauchaufsatz ist oder einen solchen aufweist.

4. Medizinischer Schlauch (10) nach einem der vorangegangenen Ansprüche, wobei die Aufnahmestruktur (3) als wenigstens eine um die Haltevorrichtung (1) oder wenigstens einen Abschnitt hiervon umlaufende Aussparung ausgestaltet ist, oder wenigstens eine solche aufweist.

5. Medizinischer Schlauch (10) nach einem der vorangegangenen Ansprüche, wobei die Aufnahmestruktur (3) als wenigstens eine um die Haltevorrichtung (1) oder wenigstens einen Abschnitt hiervon umlaufende Erhebung ausgestaltet ist oder wenigstens eine solche aufweist.

6. Medizinischer Schlauch (10) nach einem der vorangegangenen Ansprüche, wobei die Haltevorrichtung (1) zusammen mit der Schlauchklemme (4) sterilisiert ist.

7. Medizinischer Schlauch (10) nach einem der vorangegangenen Ansprüche, wobei die Haltevorrichtung (1) oder wenigstens ein Abschnitt hiervon aus einem ersten Material hergestellt ist oder ein solches aufweist, wobei der Schlauch (10) oder wenigstens ein Abschnitt des Schlauchs (10) aus einem zweiten Material hergestellt ist oder ein solches aufweist, und wobei das erste Material sich vom zweiten Material unterscheidet.

8. Medizinischer Schlauch (10) nach einem der vorangegangenen Ansprüche, ausgestaltet als extrakorporaler Blutkreislauf oder als ein Abschnitt hiervon.

9. Verfahren zum Sterilisieren eines medizinischen Schlauchs (10) nach einem der vorangegangenen Ansprüche, welcher wenigstens eine Schlauchklemme (4) aufweist, welche wenigstens einen Klemmabschnitt (7a, 7b) zum Verändern eines Lumenquerschnitts des medizinischen Schlauchs (10) hat, und welcher wenigstens eine Haltevorrichtung (1) mit einer Aufnahmestruktur (3) zum lösbaren Aufnehmen wenigstens eines Abschnitts der Schlauchklemme (4) aufweist, mit den Schritten:
- Verbringen der Schlauchklemme (4) in eine Parkposition an der Haltevorrichtung (1), in welcher der Abschnitt der Schlauchklemme (4) in oder an der Aufnahmestruktur (3) angeordnet ist; und
- gemeinsames Sterilisieren von Schlauch (10) und der in die Parkposition verbrachten Schlauchklemme (4).

10. Verfahren nach Anspruch 9, mit dem Schritt:
- Verbringen wenigstens eines Klemmabschnitts (7a, 7b) in oder an die Aufnahmestruktur (3) vor oder zum Sterilisieren des medizinischen Schlauchs (10).

## Claims

1. A medical tube (10) comprising at least one holding apparatus (1) with a tube clamp (4),
the holding apparatus (1) being configured to hold the tube clamp (4) on the medical tube (10),
the tube clamp (4) comprising at least one clamping portion (7a, 7b) for altering a lumen cross-section of the medical tube (10) during use,
the holding apparatus (1) comprising at least one receiving structure (3) for detachably receiving at least a portion of the tube clamp (4),
the portion of the tube clamp (4), which is detachably received by the receiving structure (3), being or comprising at least one aforesaid clamping portion (7a, 7b),
**characterized in that**
the holding apparatus (1) and/or its receiving structure (3) is/are provided to prevent a modification in the lumen cross-section of the tube (10), in particular in its flexible line portion, by means of the tube clamp (4), as long as the tube clamp (4) is in the park position in which the portion of the tube clamp (4) is arranged in or on the receiving structure (3).

2. The medical tube (10) according to claim 1, wherein the holding apparatus (1) is a tube connector or comprises such one.

3. The medical tube (10) according to claim 1, wherein the holding apparatus (1) is a tube attachment or comprises such one.

4. The medical tube (10) according to anyone of the preceding claims, wherein the receiving structure (3) is configured as at least one recess encircling the holding apparatus (1) or at least a portion thereof, or comprises at least such a recess.

5. The medical tube (10) according to anyone of the preceding claims, wherein the receiving structure (3) is configured as at least one protrusion encircling the holding apparatus (1) or at least a portion thereof, or comprises at least such a protrusion.

6. The medical tube (10) according to anyone of the preceding claims, wherein the holding apparatus (1) is sterilized jointly with the tube clamp (4).

7. The medical tube (10) according to anyone of the preceding claims, wherein the holding apparatus (1) or at least a portion thereof is made of or comprises a first material, wherein the tube (10) or at least a portion of the tube (10) is made of or comprises a second material, and wherein the first material is different from the second material.

8. The medical tube (10) according to anyone of the preceding claims, configured as an extracorporeal blood circuit or as a portion thereof.

9. A method for sterilizing a medical tube (10) according to anyone of the preceding claims, which comprises at least one tube clamp (4) having at least one clamping portion (7a, 7b) for modifying one lumen cross-section of the medical tube (10), and having at least one holding apparatus (1) with a receiving structure (3) for detachably receiving at least one portion of the tube clamp (4), comprising the steps of:
- placing the tube clamp (4) into a park position on the holding apparatus (1), in which the portion of the tube clamp (4) is arranged in or on the receiving structure (3) ; and
- jointly sterilizing the tube (10) and the tube clamp (4) placed in the park position.

10. The method according claim 9 comprising the step of:
- placing at least one clamping portion (7a, 7b) in or on the receiving structure (3) before or to sterilize the medical tube (10).

## Revendications

1. Un tube médical (10) comprenant au moins un dispositif de maintien (1) avec un collier de serrage (4),
Le dispositif de maintien (1) étant conçu pour maintenir le collier de serrage (4) sur le tube médical (10),
le collier de serrage (4) comprenant au moins une section de serrage (7a, 7b) destinée à modifier une section transversale luminale du tube médical (10) lors de son usage,
le dispositif de maintien (1) comprenant au moins une structure de réception (3) pour recevoir de manière détachable au moins une section du collier de serrage (4), la section du collier de serrage (4), accueillie de manière détachable par la structure de réception (3), étant ou comprenant au moins une section de serrage (7a, 7b) précitée.
**caractérisé en ce que**
le dispositif de maintien (1) et/ou sa structure de réception (3) est/sont prévu(e) (s) (es) pour empêcher une modification de la section transversale luminale du tube (10), en particulier dans sa section de conduit flexible, au moyen du collier de serrage (4), tant que le collier de serrage (4) se trouve en position de stationnement, position dans laquelle la section du collier de serrage (4) est agencée dans ou sur la structure de réception (3).

2. Le tube médical (10) selon la première revendication, où le dispositif de maintien (1) est un raccord de tube ou en comprend un.

3. Le tube médical (10) selon la première revendication, où le dispositif de maintien (1) est une fixation de tube ou en comprend une.

4. Le tube médical (10) selon l'une quelconque des revendications précédentes, où la structure de réception (3) est configurée sous la forme d'au moins un évidement entourant le dispositif de maintien (1) ou au moins une section de celui-ci, ou comprend au moins un tel évidement.

5. Le tube médical (10) selon l'une quelconque des revendications précédentes, où la structure de réception (3) est configurée sous la forme d'au moins une saillie entourant le dispositif de maintien (1) ou au moins une section de celui-ci, ou comprend au moins une telle saillie.

6. Le tube médical (10) selon l'une quelconque des revendications précédentes, où le dispositif de maintien (1) est stérilisé conjointement avec le collier de serrage (4).

7. Le tube médical (10) selon l'une quelconque des revendications précédentes, où le dispositif de maintien (1), ou au moins une partie de celui-ci, est constitué(e) d'un premier matériau, ou en comprend un, et où le tube (10), ou au moins une section du tube (10), est constitué(e) d'un second matériau, ou en comprend un, et où le premier matériau est différent du second matériau.

8. Le tube médical (10) selon l'une des revendications précédentes, conçu comme un circuit sanguin extracorporel ou comme une section de celui-ci.

9. Un procédé de stérilisation d'un tube médical (10) selon l'une quelconque des revendications précédentes, qui comprend au moins un collier de serrage (4) ayant au moins une section de serrage (7a, 7b) pour modifier une section transversale luminale du tube médical (10), et ayant au moins un dispositif de maintien (1) doté d'une structure de réception (3) pour recevoir de manière détachable au moins une section du collier de serrage (4), comprenant les étapes qui consistent à:
- placer le collier de serrage (4) en position de stationnement sur le dispositif de maintien (1), position dans laquelle la section du collier de serrage (4) est agencée dans ou sur la structure de réception (3); et
- stériliser conjointement le tube (10) et le collier de serrage (4) placé en position de stationnement.

10. Le procédé selon la revendication 9 comprenant l'étape qui consiste à:
- placer au moins une section de serrage (7a, 7b) dans ou sur la structure de réception (3) avant de ou pour stériliser le tube médical (10).
